# EUROPÄISCHE PATENTSCHRIFT

(11) **EP 1 060 177 B1**
(45) Veröffentlichungstag und Bekanntmachung des Hinweises auf die Patenterteilung: **09.08.2006**
(21) Anmeldenummer: 98966382.8
(22) Anmeldetag: 18.12.1998
(51) Int. Cl.: C07D 405/14, A61K 31/505

(54) **VERFAHREN ZUR HERSTELLUNG VON DOXAZOSIN-MESYLAT IN EINER ALS FORM A BEZEICHNETEN KRISTALLMODIFIKATION UND EIN ZWISCHENPRODUKT FÜR DEREN HERSTELLUNG**
METHOD FOR PRODUCING DOXAZODIN MESYLATE IN A CRYSTAL MODIFICATION DESIGNATED AS FORM A AND INTERMEDIATE PRODUCT FOR PRODUCING THE SAME
PROCEDE DE FABRICATION DE MESYLATE DE DOXAZOSINE SELON UNE MODIFICATION CRISTALLINE DESIGNEE PAR FORME A ET PRODUIT INTERMEDIAIRE POUR CETTE FABRICATION

(30) Priorität: 06.01.1998 DE 19800214
(43) Veröffentlichungstag der Anmeldung: 20.12.2000
(73) Patentinhaber: Chemische Fabrik Berg GmbH, 06749 Bitterfeld (DE)
(72) Erfinder: KLEIN, Peter, D-67134 Birkenheide (DE); THYES, Marco, D-67061 Ludwigshafen (DE); HLIX, Dieter, D-67229 Grosskarlbach (DE)
(74) Vertreter: Graf von Stosch, Andreas
(86) Internationale Anmeldenummer: PCT/EP1998/008360
(87) Internationale Veröffentlichungsnummer: WO 1999/035143

(56) Entgegenhaltungen:
- EP-A- 0 459 666
- EP-A- 0 848 001
- EP-A- 0 849 264
- EP-A- 0 849 265
- EP-A- 0 849 266
- WO-A-94/09783
- WO-A-96/39406
- US-A- 4 188 390
- GRCMAN M ET AL: "STUDY OF POLYMORPHISM OF 1-(4-AMINO-6,7-DIMETHOXY-2-QUINAZOLINYL) -4- 2,3-DIHYDRO-1,4-BENZODIOXIN-2-YL)CARBONYL- PIPERAZINE MONOMETHANESULFONATE" FARMACEVTSKI VESTNIK, Bd. 48, 1997, Seite 292/293 XP002058927
- LYING,X. ET AL.: "Studies of Polymorphism of Doxazosin " CHINES.J.MED., Bd. 5, Nr. 4, 1995, Seite 266-70 XP002101846 in der Anmeldung erwähnt
- GIRON ET AL: "Thermal analysis and calorimetric methods in the characterisation of polymorphs and solvates" THERMOCHIMICA ACTA, Bd. 248, Nr. 1/02, 1. Januar 1995, Seiten 1-59, XP002081715
- THRELFALL,T.L.: "Analysis of Organic Polymorphs " ANALYST, Bd. 120, Oktober 1995, Seiten 2435-22460, XP002101807 LONDON

## Beschreibung

Die vorliegende Erfindung betrifft ein Verfahren zur Herstellung von Doxazosin-Mesylat in einer als Form A bezeichneten Kristallmodifikation und ein Zwischenprodukt, über welches dieses Verfahren läuft.

Doxazosin (= 4-Amino-2-[4-(1,4-benzodioxan-2-carbonyl)piperazin-1-yl]-6,7-dimethoxychinazolin) ist eine bekannte Substanz (Merck-Index 12. Auflage 1996, Nr. 3489), die den Blutdruck senkt. Die Substanz wird überwiegend in der Form des Monomesylats verwendet, welches in kristalliner Form nach den im Chinese Journal of Medicinal Chemistry 5(4), 266 - 270 (1995) gemachten Angaben bislang in 3 Kristallmodifikationen vorkommt. Die verschiedenen Modifikationen werden in der genannten Literaturstelle als Modifikationen A, B und C bezeichnet. Modifikation A wird bei der Umkristallisation von Doxazosin-Mesylat aus Ethanol gewonnen. Die Modifikationen B und C entstehen bei der Umkristallisation von Doxazosin-Mesylat aus Chloroform bzw. Wasser. Es sei darauf hingewiesen, daß im Chinese Journal of Medicinal Chemistry zwar lediglich von Doxazosin gesprochen wird. Nach den publizierten Daten muß es sich jedoch um das Doxazosin-Mesylat handeln.

Obwohl das Doxazosin meistens als Monomesylat verwendet wird, ist die Herstellung dieses Salzes bislang nicht beschrieben worden. Was die bekannten Modifikationen des Doxazosin-Mesylats angeht, so hat es sich gezeigt, daß sich das Mesylat in der Siedehitze auch in der 75-fachen Menge Chloroform nicht löst. Das nach Abkühlen der Suspension isolierte Produkt enthält Chloroform - ein aus toxikologischer Sicht bedenkliches Lösungsmittel -, das sich nur schwer und auch nicht ohne Beeinträchtigung des Wirkstoffes entfernen läßt. In Wasser ist das Mesylat zwar besser löslich, das beim Abkühlen der Lösung anfallende Produkt läßt sich aber nur schwer isolieren. Weiterhin ist die Ausbeute schlecht und das Material erweist sich als röntgenamorph. In Ethanol löst sich das Mesylat auch in der 350-fachen Menge des Lösungsmittels in der Siedehitze nicht. Heißfiltration der Suspension liefert als Filterrückstand in geringer Ausbeute die beschriebene Ethanol-Modifikation A. Abkühlen des Filtrats führt in nur mäßiger Ausbeute zu verunreinigter A-Modifikation.

Es ist also nicht nur die Herstellung des Doxazosin-Mesylats bisher nicht beschrieben worden, sondern es gibt auch keine im technischen Maßstab verwendbare Methode, um Doxazosin-Mesylat gezielt in einer bestimmten Modifikation zu erhalten. Von den beschriebenen Modifikationen kommen die Modifikationen B und C auf Grund der oben angegebenen Eigenschaften für eine Verfahrensentwicklung nicht in Betracht. Lediglich die Modifikation A ist von ihren Eigenschaften her hierfür geeignet.

Es wurde nun ein im technischen Maßstab verwendbares Verfahren zur Herstellung von Doxazosin-Mesylat in der Kristallmodifikation A gefunden.

Gegenstand der Erfindung ist ein Verfahren zur Herstellung von Doxazosin-Mesylat in der Modifikation A, welches darin besteht, daß man Doxazosin mit Methansulfonsäure in Methanol löst, die so erhaltene Lösung gegebenenfalls von einer Trübung befreit und die dabei anfallende, klare Lösung so lange rührt, bis kein Niederschlag mehr ausfällt, abtrennt, mit Methanol wäscht, den Niederschlag in feuchtem Zustand in Ethanol erhitzt und nach dem Abkühlen das entstandene Produkt isoliert.

Zur Umsetzung von Doxazosin mit Methansulfonsäure werden die beiden Substanzen im Molverhältnis von etwa 1:1 eingesetzt. Vorzugsweise wird ein geringer molarer Überschuß an der Sulfonsäure verwendet (bis etwa 10 %).

Falls die Zeit zwischen dem Erhalt einer Lösung durch Zugabe von Methansulfonsäure zum Doxazosin und dem Erscheinen eines Niederschlags nicht für eine Filtration ausreicht - beispielsweise wenn die Umsetzung im technischen Maßstab durchgeführt werden soll - kann man die Zeit für die Filtration dadurch verlängern, daß man dem für die Umsetzung verwendeten Methanol ein aprotisches, polares organisches Lösungsmittel zusetzt.

Als aprotische, polare organische Lösungsmittel eignen sich in diesem Fall beispielsweise N,N-Dimethylformamid und insbesondere N-Methyl-2-pyrrolidon. Das Verhältnis von Doxazosin zu Methanol (Gewicht/Volumen) bzw. das Verhältnis von Doxazosin zu Methanol zu dem aprotischen, polaren organischen Lösungsmittel (Gewicht/Volumen/Volumen) beträgt etwa 1 : (5 bis 15), vorzugsweise etwa 1 : (8 bis 12) bzw. etwa 1 : (5 bis 15) : (1,5 bis 4), vorzugsweise etwa 1 : (8 bis 12) : (2 bis 3).

Soll bei dem neuen Verfahren die nach der Zugabe der Methansulfonsäure erhaltene Lösung durch Filtrieren von ggf. vorhandenen Fremdpartikeln befreit werden, so wird bevorzugt in der ersten Reaktionsstufe mit dem Lösungsmittelgemisch aprotisches, polares organisches Lösungsmittel/Methanol gearbeitet. Grund ist, daß in diesem Falle, wie schon erwähnt, die Zeitspanne zwischen Erhalt der Lösung und Bildung erster Kristalle größer ist als bei alleiniger Verwendung von Methanol als Lösungsmittel. Wird in der ersten Reaktionsstufe des neuen Verfahrens eine Filtration der nach der Zugabe der Methansulfonsäure erhaltenen Lösung gewünscht, so besteht eine besonders bevorzugte Vorgehensweise darin, daß in dem Lösungsmittelgemisch aus aprotischem, polarem organischem Lösungsmittel gearbeitet wird und darüber hinaus ein Teil des Methanols erst nach der Filtration zugegeben wird.

Der nach dem Zusammengeben von Doxazosin und Methansulfonsäure entstehende Niederschlag stellt eine neue Modifikation des Doxazosin-Mesylats dar, die hier als Modifikation D bezeichnet wird. Diese Modifikation ist ebenfalls Gegenstand der Erfindung. Sie ist insbesondere charakterisiert durch Hauptlinien im Debye-Scherrer Röntgen-Diffraktogramm bei folgenden, in Winkelgrad angegebenen Werten von 2 Theta: 5,72 ± 0,2°; 11,10 ± 0,2°; 11,46 ± 0,2°; 14,14 ± 0,2°; 17,01 ± 0,2°; 17,78 ± 0,2°; 18,33 ± 0,2°; 20,73 ± 0,2°; 21,70 ± 0,2°; 23,12 ± 0,2°; 24,28 ± 0,2°; 26,58 ± 0,2°.

Das Doxazosin-Mesylat (Modifikation D) wird nach seiner Abtrennung mit Methanol gewaschen und das lösungsmittelfeuchte Produkt (Feuchtigkeitsgehalt etwa 10 - 60 %, vorzugsweise 25-50%) zur Modifikation A des Doxazosin-Mesylats weiterverarbeitet.

Die Umwandlung der feuchten Modifikation D erfolgt auf einfache Weise durch Erwärmen in Ethanol. Dabei wird vorzugsweise unter Erwärmen auf Rückfluß gearbeitet. Die Menge des verwendeten Ethanols ist so zu bemessen, daß bei der Umwandlung stets eine Suspension vorliegt. Sie beläuft sich vorzugsweise auf etwa das Zehnfache der eingesetzten Menge an feuchter Modifikation D, berechnet auf deren Trockengewicht, d.h. für 10 g Trockensubstanz werden 100 mL Ethanol verwendet.

Das neue Verfahren liefert Doxazosin-Mesylat in der Modifikation A in einer Gesamtausbeute von über 85 %. Die Reinheit der nach dem neuen Verfahren erhaltenen Modifikation A ist ausgezeichnet. Ein wesentlicher Vorteil des neuen Verfahrens besteht auch darin, daß nach der Zugabe der Methansulfonsäure zum Doxazosin eine Lösung entsteht. Dadurch ist es möglich, durch Filtrieren gegebenenfalls vorhandene Fremdpartikel zu entfernen.

### Beispiel 1

### Herstellung von Doxazosin-Mesylat der Kristallmodifikation D

### Verfahren 1

In einem 1-L-Dreihalsrundkolben wurde ein Gemisch aus 63,2 g Doxazosin, 125 mL N-Methyl-2-pyrrolidon und 500 mL Methanol unter Rühren mit 14,1 g wasserfreier Methansulfonsäure versetzt. Dabei stieg die Innentemperatur auf 30°C an, und es entstand eine Lösung. Nach beendeter Zugabe der Methansulfonsäure wurde der Reaktionsansatz in einen zweiten 1-L-Dreihalsrundkolben filtriert. Der Filter wurde mit 85 mL Methanol nachgewaschen und die vereinigten Filtrate 5 h gerührt. Nach beendeter Rührzeit wurde der entstandene Niederschlag abgesaugt und 3x mit je 25 mL Methanol gewaschen. Man erhielt 125 g feuchtes Doxazosin-Mesylat (Modifikation D). Das entspricht 70,4 g Trockensubstanz und einer Ausbeute von 91,8 %.

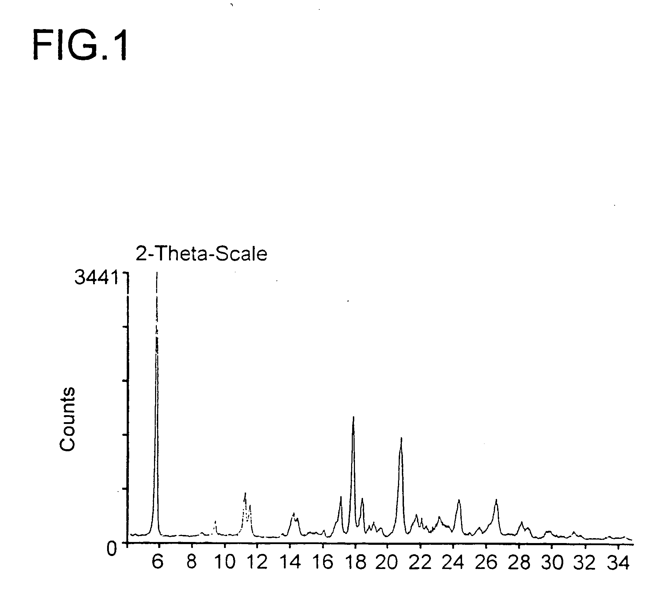

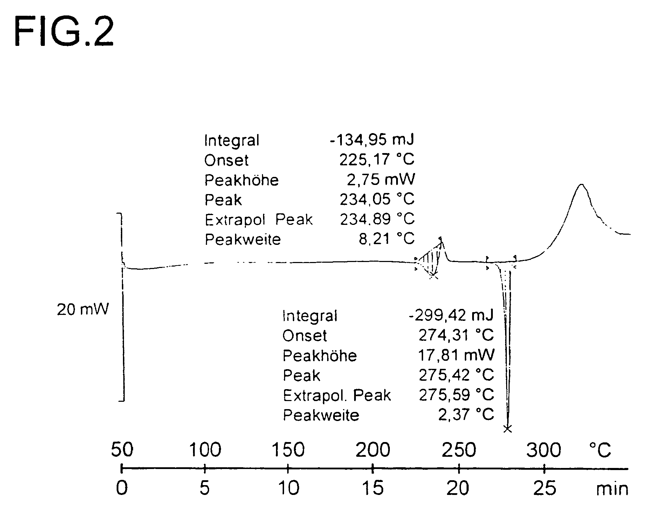

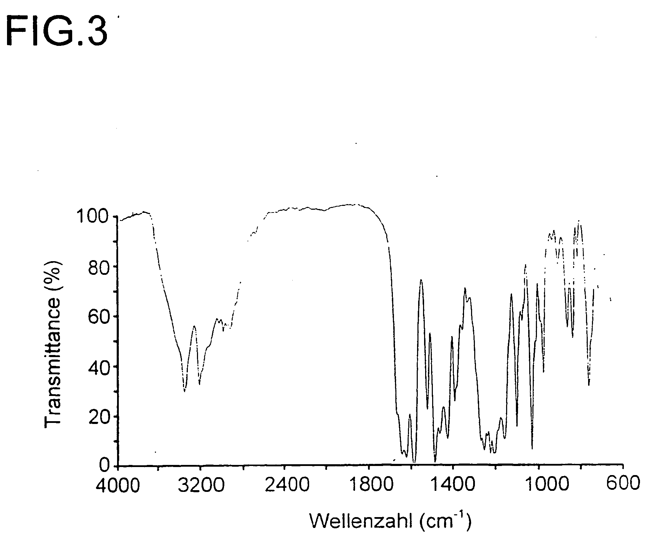

Die Modifikation D des Doxazosin-Mesylats wurde durch das Debye-Scherrer Röntgendiffraktogramm, durch das Differential Scanning Thermogramm und durch das IR-Spektrum charakterisiert (vgl. Abb. 1 - 3; für 2-Theta-Werte der Hauptlinien im Diffraktogramm siehe oben). Alle Daten wurden an im Vakuum getrocknetem Material erhoben.

### Verfahren 2

In einem 500-mL-Dreihalsrundkolben wurde ein Gemisch aus 27,1 g Doxazosin, 54 mL N-Methyl-2-pyrrolidon und 250 mL Methanol unter Rühren mit 6,1 g wasserfreier Methansulfonsäure versetzt. Dabei stieg die Innentemperatur auf 30°C an, und es entstand eine Lösung. Nach beendeter Zugabe der Methansulfonsäure wurde der Reaktionsansatz in einen zweiten 500-mL-Dreihalsrundkolben filtriert und das Filtrat 5 h gerührt. Nach beendeter Rührzeit wurde der entstandene Niederschlag abgesaugt und 2x mit je 50 mL Methanol gewaschen. Man erhielt 45 g feuchtes Doxazosin-Mesylat (Modifikation D). Das entspricht 28,5 g Trockensubstanz und einer Ausbeute von 86,7 %.

### Verfahren 3

In einem 2-L-Dreihalsrundkolben wurde ein Gemisch aus 79,0 g Doxazosin und 800 mL Methanol unter Rühren mit 17,7 g wasserfreier Methansulfonsäure versetzt. Dabei stieg die Innentemperatur auf 30°C an und es entstand eine Lösung. Nach beendeter Zugabe der Methansulfonsäure wurde noch 5 h nachgerührt. Dann wurde der entstandene Niederschlag abgesaugt und 3x mit je 50 mL Methanol gewaschen. Man erhielt 141,8 g feuchtes Doxazosin-Mesylat (Modifikation D). Das entspricht 89,2 g Trockensubstanz und einer Ausbeute von 93,1 %.

### Beispiel 2

### Umwandlung der feuchten Modifikation D des Doxazosin-Mesylats in die Modifikation A

120 g des gemäß Beispiel 1, Verfahren 1 erhaltenen, feuchten Produkts wurden in einem 1-L-Dreihalsrundkolben in 700 ml Ethanol aufgenommen. Die Mischung wurde 3 h unter Rühren bei Rückflußtemperatur gehalten (durchweg eine Lösung) und anschließend auf Raumtemperatur abgekühlt. Der ungelöste Feststoff wurde abgesaugt, 2x mit je 25 mL Ethanol gewaschen und im Vakuum bei 75°C getrocknet. Es wurden 63,9 g Doxazosin-Mesylat in der Form A erhalten. Das entspricht einer Ausbeute von 94,2 % bezogen auf die eingesetzte Menge Modifikation D.

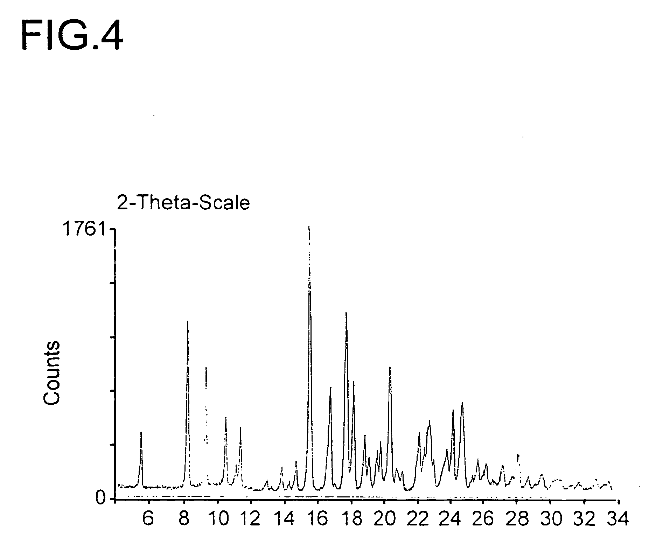

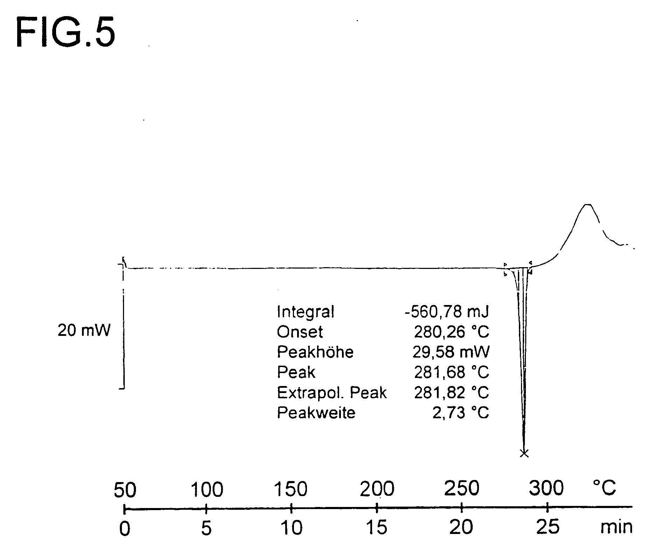

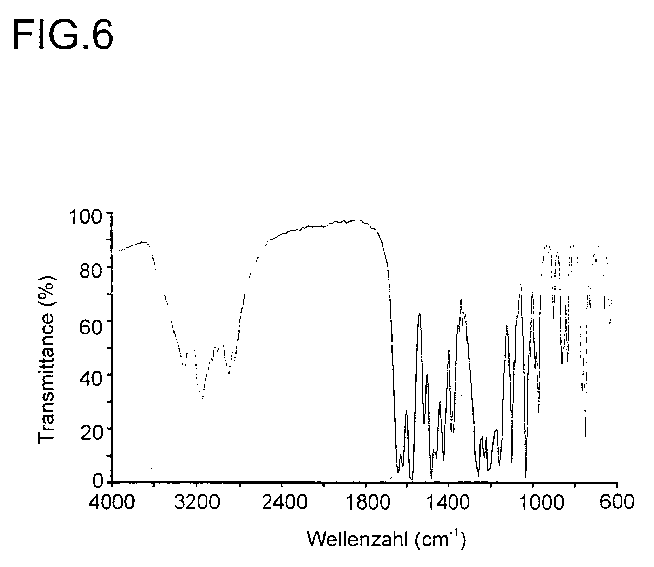

Für das Debye-Scherrer Röntgendiffraktogramm, das Differential Scanning Thermogramm und das IR-Spektrum der Form A des Doxazosin-Mesylats siehe Abb. 4 - 6.

## Patentansprüche

1. Verfahren zur Herstellung von Doxazosin-Mesylat in der Modifikation A, das charakterisiert ist durch Hauptlinien im Debye-Scherrer Röntgen-Diffraktogramm gemäß Fig. 4, **dadurch gekennzeichnet, dass** man Doxazosin mit Methansulfonsäure in Methanol löst, die so erhaltene Lösung gegebenenfalls von einer Trübung befreit und die dabei anfallende, klare Lösung so lange rührt, bis kein Niederschlag mehr ausfällt, abtrennt, mit Methanol wäscht, den Niederschlag in feuchtem Zustand in Ethanol erhitzt und nach dem Abkühlen das entstandene Produkt isoliert.

2. Verfahren gemäß Anspruch 1, **dadurch gekennzeichnet, dass** man in der 1. Reaktionsstufe statt Methanol ein Gemisch aus einem aprotischen, polaren organischen Lösungsmittel und Methanol verwendet.

3. Modifikation D von Doxazosin-Mesylat, wobei dieses charakterisiert ist durch Hauptlinien im Debye-Scherrer Röntgen-Diffraktogramm bei folgenden, in Winkelgrad angegebenen Werten von 2 Theta: 5,72 ± 0,2°; 11,10 ± 0,2°; 11,46 ± 0,2°; 14,14 ± 0,2°; 17,01 ± 0,2°; 17,78 ± 0,2°; 18,33 ± 0,2°; 20,73 ± 0,2°; 21,70 ± 0,2°; 23,12 ± 0,2°; 24,28 ± 0,2°; 26,58 ± 0,2°.

## Claims

1. Method for manufacturing the modification A of doxazosin mesylate, which is **characterised by** main lines in the Debye-Scherrer x-ray diffractogram according to Figure. 4, **characterised in that** doxazosin is dissolved with methane sulphonic acid in methanol, any cloudiness removed from the solution so obtained and the resulting, clear solution stirred until there is no more precipitation, separated, washed with methanol, the precipitation heated in the moist state in methanol and the resulting product isolated after cooling.

2. Method according to Claim 1, **characterised in that** a mixture of an aprotic, polar organic solvent and methanol is used instead of methanol in the first stage of the reaction.

3. Modification D of doxazosin mesylate, where this is **characterised by** main lines in the Debye-Scherrer x-ray diffractogram at the following values of 2 theta given in angular degrees: 5.72 ± 0.2°; 11.10 ± 0.2°; 11.46 ± 0.2°; 14.14 ± 0.2°; 17.01 ± 0.2°; 17.78 ± 0.2°; 18.33 ± 0.2°; 20.73 ± 0.2°; 21.70 ± 0.2°; 23.12 ± 0.2°; 24.28 ± 0.2°; 26.58 ± 0.2°.

## Revendications

1. Procédé pour la préparation de mésylate de doxazosine dans la modification A, qui est **caractérisée par** les pics sur le diffractogramme de rayons X de Debye-Scherrer selon la figure 4, **caractérisé en ce que** l'on dissout la doxazosine avec de l'acide méthane sulfonique dans le méthanol, **en ce que** la solution ainsi obtenue est éventuellement débarrassée de sa turbidité et **en ce que** la solution limpide obtenue, agitée jusqu'à ce qu'il n'y ait plus aucun précipité, est séparée, lavée au méthanol, le précipité à l'état humide est chauffé dans l'éthanol et, après le refroidissement, le produit obtenu est isolé.

2. Procédé selon la revendication 1, **caractérisé en ce que**, dans la première étape de réaction, à la place du méthanol, on utilise un mélange d'un solvant organique polaire aprotique et de méthanol.

3. Modification D de mésylate de doxazosine, dans laquelle celui-ci est **caractérisé par** les pics sur le diffractogramme de rayons X de Debye-Scherrer, ayant les valeurs suivantes indiquées en degrés angulaires de 2 thêta : 5,72 ± 0,2° ; 11,10 ± 0,2° ; 11,46 ± 0,2° ; 14,14 ± 0,2° ; 17,01 ± 0,2° ; 17,78 ± 0,2° ; 18,33 ± 0,2° ; 20,73 ± 0,2° ; 21,70 ± 0,2° ; 23,12 ± 0,2° ; 24,28 ± 0,2° ; 26,58 ± 0,2°.
